# EUROPEAN PATENT APPLICATION

(11) **EP 1 719 514 A1**
(43) Date of publication of application: **08.11.2006**
(21) Application number: 05704116.2
(22) Date of filing: 26.01.2005
(51) Int. Cl.: A61K 31/704, A61P 25/04

(54) **DRUG FOR TREATING PAIN**

(30) Priority: 26.01.2004 JP 2004017024
(71) Applicant: Ninomiya, Kozo, Setagaya-ku Tokyo 1540024 (JP); Ninomiya, Shuzo, Kyoto-shi, Kyoto 612-0031 (JP)
(72) Inventor: Ninomiya, Kozo, Setagaya-ku Tokyo 1540024 (JP); Ninomiya, Shuzo, Kyoto-shi, Kyoto 612-0031 (JP)
(74) Representative: Sternagel, Fleischer, Godemeyer & Partner
(86) International application number: PCT/JP2005/000979
(87) International publication number: WO 2005/070437

(57) **Abstract**

A medicament for therapeutic and/or prophylactic treatment of pain such as neuropathic pain that occurs after peripheral or central nerve injury, which contains ginsenoside Rb1 as an active ingredient.

SEQUENCE LISTING
<110> Shuzo Ninomiya
<120> Medicament for treatment of pain
<130> A41868M
<160> 4
<210> 1
<211> 24
<212> DNA
<213> artificial
<400> 1
agaagagcta tgagctgcct gacg 24
<210> 2
<211> 24
<212> DNA
<213> artificial
<400> 2
tacttgcgct caggaggagc actg 24
<210> 3
<211> 20
<212> DNA
<213> artificial
<400> 3
gctcgctgaa ccctgttatc 20
<210> 4
<211> 20
<212> DNA
<213> artificial
<400> 4
tcccctccaa actgggtatt 20

## Description

### Technical Field

The present invention relates to a medicament for therapeutic and/or prophylactic treatment of pain such as neuropathic pain that occurs after peripheral or central nerve injury.

### Background Art

Peripheral neuropathy causes neuropathic pain (also referred to as "neurogenic pain") such as hyperalgesia (increased response to a stimulus normally felt as painful) and allodynia (pain caused by a stimulus which does not normally cause pain), as well as hypoesthesia and hypokinesia. Neuropathic pain occurs when the primary afferent sensory neuron is damaged by a certain cause. A mechanism of onset thereof remains unknown and no effective therapy has been established. Therefore, the pain is considered as one of most agonizing pains for patients (Textbook of Pain, 3rd Edition, London, Longman Group, pp.201-224, 1994). Although the onset mechanism of neuropathic pain essentially remains unknown, various theories have been proposed (Non-patent document 1). It is considered that one of causes thereof is a plastic change in the descending pain modulatory system (noradrenalinergic nervous system/serotonergic nervous system), which starts from the brain stem and descends to the dorsal horn of the spinal cord (Clinical Neuroscience, Vol. 20, pp.1122-1125, 2002).

It has recently been being revealed that a ginseng saponin, ginsenoside Rb1, has effects of preventing and improving cerebral infarction and spinal cord injury. Wen et al. reported that, after oral administration of the red ginseng powder or intraperitoneal administration of crude ginseng saponin or ginsenoside Rb1 over one week before 5-minute transient forebrain ischemia, actions of improvement of learning and behavioral impairment and inhibition of delayed nerve cell death were observed (Acta Neuropathol (Berl), Vol. 91, pp.15-22, 1996). Lim et al. reported that, when ginsenoside Rb1 was continuously injected into the brain ventricle for 7 days immediately after loading of transient forebrain ischemia, the response latency in a passive avoidance learning experiment significantly prolonged in a dose-dependent manner compared with a control, and that delayed nerve cell death in the hippocampus CA1 region was significantly reduced (Neuroscience Research, Vol. 28, pp.191-200, 1997). Further, Nakata reported that, after intravenous administration of a crude ginseng saponin immediately after compression of the spinal cord, motor paralysis improving effect was observed (Ehime Igaku (Ehime Medical Journal), 21-1, pp.24-30, 2002). However, no report was made as for effect of ginsenoside Rb1 on neuropathic pain animal models. It is known that ginsenosides Rc, Rd, Re and Rf have analgesic effect in mice (General Pharmacology, Vol. 32, pp.653-659, 1999; Brain Research, Vol. 792, pp.218-228, 1998). However, analgesic effect of ginsenoside Rb1 has not yet been reported.

### Disclosure of the Invention

### Object to be Achieved by the Invention

An object of the present invention is to provide a medicament for therapeutic and/or prophylactic treatment of pain such as neuropathic pain that occurs after peripheral or central nerve injury.

### Means for Achieving the Object

The inventors of the present invention conducted various researches to achieve the foregoing object. As a result, they found that ginsenoside Rb1 successfully and remarkably relieved neuropathic pain, and that a medicament comprising ginsenoside Rb1 as an active ingredient was extremely useful for therapeutic and/or prophylactic treatment of pain such as neuropathic pains occurring after peripheral or central nerve injury. The present invention was achieved on the basis of the above findings.

The present invention thus provides a medicament for therapeutic and/or prophylactic treatment of pain, which comprises ginsenoside Rb1 as an active ingredient. According to preferred embodiments of the present invention, there are provided the aforementioned medicament, wherein the pain is chronic pain; the aforementioned medicament, wherein the pain is neuropathic pain; and the aforementioned medicament, wherein the neuropathic pain is pain induced by peripheral and/or central nerve injury. The present invention also provides a medicament for therapeutic and/or prophylactic treatment of pain, which is an α2A-adrenergic receptor-agonistic medicament comprising ginsenoside Rb1 as an active ingredient.

From other aspects of the present invention, there are provided use of ginsenoside Rb1 for the manufacture of the aforementioned medicament; and a method for therapeutic and/or prophylactic treatment of pain, which comprises the step of administering a therapeutically and/or prophylactically effective amount of ginsenoside Rb1 to a mammal including human. The present invention further provides a method for therapeutic and/or prophylactic treatment of pain, wherein the therapeutic and/or prophylactic treatment of pain is attained via the α2A-adrenergic receptor by administering a therapeutically and/or prophylactically effective amount of ginsenoside Rb1 to a mammal including human.

### Brief Description of the Drawings

[Fig. 1] Fig. 1 shows results indicating that hyperalgesia was significantly relieved from 3 weeks after sciatic nerve ligation by continuously administering ginsenoside Rb 1 for 4 weeks from 24 hours after the ligation.
[Fig. 2] Fig. 2 shows result indicating that allodynia was not ameliorated even by continuously administering ginsenoside Rb1 for 4 weeks from 24 hours after sciatic nerve ligation.
[Fig. 3] Fig. 3 shows results of examination of hyperalgesia suppressing effect of ginsenoside Rb1, which was performed by administering a serotonin depletor (5,7-DHT) 3 weeks after sciatic nerve ligation and performing a heat test before and after the administration.
[Fig. 4] Fig. 4 shows results of examination of hyperalgesia suppressing effect of ginsenoside Rb1, which was performed by performing a heat test 3 weeks after sciatic nerve ligation, administering a β-adrenergic receptor blocker (propranolol) on the next day and performing the heat test again one hour later.
[Fig. 5] Fig. 5 shows results of examination of hyperalgesia suppressing effect of ginsenoside Rb1, which was performed by performing a heat test 3 weeks after sciatic nerve ligation, administering an opioid receptor antagonist (naloxone) on the next day and performing the heat test again one hour later.
[Fig. 6] Fig. 6 shows results of examination of hyperalgesia suppressing effect of ginsenoside Rb1, which was performed by performing a heat test 3 weeks after sciatic nerve ligation, administering an α-adrenergic receptor blocker (phentolamine) on the next day and performing the heat test again one hour later.
[Fig. 7] Fig. 7 shows expression of mRNA of the α2A-adrenergic receptor in the spinal cord by ginsenoside Rb1 3 weeks after sciatic nerve ligation.
[Fig. 8] Fig. 8 shows expression of the α2A-adrenergic receptor protein in the spinal cord by ginsenoside Rb 1 3 weeks after sciatic nerve ligation.

### Best Mode for Carrying out the Invention

Ginsenoside Rb1 is a class of ginseng saponin which is known to be useful for prevention, treatment or therapy of brain diseases and prevention, treatment or therapy of neurotrauma as described in, for example, International Patent Publications WO00/37481, WO00/48608, WO01/15717, WO01/92289 and the like. This substance is readily available for those skilled in the art. A method for screening for substances having an action similar to that of ginsenoside Rb1 (ginsenoside Rb1-like substances) is known (patent application in Japan, Applicant: Masahiro Sakanaka, Title of the Invention: "A method for screening for ginsenoside Rb1-like substances"). Accordingly, a substance screened by this method can also be used as an active ingredient of the medicament of the present invention. The term "ginsenoside Rb1" used in the present specification encompasses any substances screened by the aforementioned screening method (e.g., glycosides and the like) in addition to the naturally-derived ginsenoside Rb1, and should not be construed in any limitative sense. Further, substances in the form of salts, or substances in the form of hydrates or solvates can also be used as an active ingredient of the medicament of the present invention.

The medicament of the present invention is useful for therapeutic and/or prophylactic treatment of pain. Pain is generally defined as unpleasant sense and emotional experience associated with substantial or potential tissue damage or represented with a term of such damage (International Association for the Study of Pain (IASP), 1994), and includes physiological pain (acute nociceptive pain), pathological sustained pain (tissue damage/inflammation-induced pain, neuropathic pain) and psychogenic pain. The medicament of the present invention can be applied to any of the aforementioned types of pain, which include chronic pain as a preferred object to be treated. A further preferred example of pain to be treated includes neuropathic pain.

Neuropathic pain is intractable pain resulted from dysfunction of the peripheral or central nervous system and clinically accounts for most part of chronic pain. Typical examples of the major symptoms include three types of pain, "hyperalgesia", which is an increased response to a stimulus normally felt as painful, "allodynia", which is pain caused by a stimulus that does not normally cause pain, and "sustained spontaneous pain". The medicament of the present invention can be applied to any of these symptoms, and "hyperalgesia" and "sustained spontaneous pain" are preferred symptoms to be treated, and a particularly preferred symptom to be treated is "hyperalgesia".

Neuropathic pain is often resistant to non-steroidal anti-inflammatory drugs (NSAIDs) such as indomethacin,and analgesics available including opioid analgesics such as morphine, and therapeutic and prophylactic treatments thereof often become difficult as intractable pain. The medicament of the present invention can exhibit an excellent analgesic effect also on such neuropathic pain resistant to analgesics available. For example, neuropathic pain that occurs after peripheral nerve injury and central nerve injury resulting from cerebral infarction, spinal cord injury or the like, neuropathic pain resulting from cancer and the like are preferred symptoms to be treated with the medicament of the present invention. However, types of pain to be treated with the medicament of the present invention are not limited to those specifically explained above.

It can be readily confirmed by those skilled in the art that the medicament of present invention has superior effect on pain by using the methods specifically explained in the examples of the present specification. As experimental animal models associated with pain, thermal stimulus and mechanical stimulus models can be utilized as physiological pain models, and inflammatory pain models (chemical stimulus) and neuropathic pain models can be utilized as pathological pain models. As the neuropathic pain models, chronic constriction injury (CCI) model, spinal nerve ligation (Chung) model, spared nerve injury model and the like can be utilized, but the models are not limited to these examples.

As the medicament of the present invention, ginsenoside Rb1, per se, may be administered. The medicament can be preferably administered as a pharmaceutical composition for oral or parenteral administration, which can be prepared by those skilled in the art according to a well-known method. The medicament of the present invention can generally be prepared as a pharmaceutical composition for parenteral administration, and the composition can be prepared in the form of, for example, injection, drip infusion, suppository, inhalation, eye drop, nasal drop, ointment, transdermal preparation, transmucosal preparation, cream, patch or the like, preferably, injection or drip infusion for intravenous administration. Examples of the pharmaceutical composition suitable for oral administration include ginsenoside Rb1-like substances suitable for oral administration or ginsenoside Rb1-like substances prepared as pro-drugs, which are prepared as a medicament in the form of, for example, tablet, capsule, powder, subtilized granule, granule, solution, syrup or the like.

The aforementioned pharmaceutical composition can be prepared by adding pharmacologically and pharmaceutically acceptable additives. Examples of the pharmacologically and pharmaceutically acceptable additives include, for example, excipients, disintegrating agents or aids, binders, lubricants, coating agents, dyes, diluents, vehicles, dissolving agents or aids, isotonic agents, pH modifiers, stabilizers, propellants, tackifiers and the like. The aforementioned pharmaceutical composition may be mixed with one or more types of other medicaments such as analgesics for treatment of pain. Use of ginsenoside Rb1 as a medicament is described in, for example, Japanese Patent Unexamined Publication (Kokai) Nos. 2002-53467, 2000-191539, 2000-302798 and the like, and the medicaments in the forms described in these patent publications can be suitably used as the medicament of the present invention.

Doses of the medicament of the present invention are not particularly limited and can be appropriately increased or decreased depending on various factors that should usually be considered, such as type of pain, body weight, age and symptom of a patient and route of administration. In general, for oral administration, a daily dose for an adult may be in the range of about 0.01 to 1000 mg in terms of the weight of ginsenoside Rb1 as an active ingredient.

### Examples

The present invention will be explained more specifically with reference to the following examples. However, the scope of the present invention is not limited to these examples.
Example 1
A. Materials and methods
(1) Preparation of sciatic nerve ligation injury model
A sciatic nerve ligation injury model was prepared by modifying the neuropathic pain model published by Bennett et al. according to the method of Takeba (Pain, Vol. 33, pp.87-107, 1988; Ehime Igaku (Ehime Medical Journal), Vol. 21, pp.192-206, 2002). Under anesthesia by inhalation of 3% halothane and nitrous oxide, bilateral sciatic nerves of 8- to 12-week old female Wistar rats (Clea Japan, Inc., Osaka) were exposed and ligated at the central level of the femur along with a 27-gauge injection needle on the both sides by using a 4-0 polyglycholic acid suture at 4 sites at intervals of 1 mm for both sides. Then, the injection needle was removed to prepare a sciatic nerve ligation injury model. The ligation was attained to such a degree that the crural muscles slightly contractable by a stimulus given to the nerve, and care was taken not to cause injury in the axon by the ligation operation itself.

### (2) Method for intravenously administering ginsenoside Rb 1

### (a) Attachment of continuous injection pump

Alzet Mini-Osmotic Pump Model 2004 (DURECT Corporation, Cupertino, USA) was used as a continuous injection pump. The right external jugular vein was exposed, a silicon tube (HELIX MEDICAL, Carpinteria, USA) connected to the pump was inserted by 2.5 cm in the vein and fixed, and the pump was subcutaneously placed on the back of rats. Before use, the silicon tube was injected with heparin, remained overnight and washed with saline. (b) Intravenous administration of ginsenoside Rb1
Ginsenoside Rb1 was dissolved in saline. In the continuous injection pump, each of the followings was filled beforehand.
(i) Ginsenoside Rb1 12 µg/day × 28 days,
(ii) Ginsenoside Rb1 60 µg/day × 28 days
(iii) Saline (Otsuka)

A solution of ginsenoside Rb1 (0.10 or 0.50 µg/µl) in a volume of 120 µl or an equivalent volume of saline was intravenously administered by one-shot to rats 24 hours after the sciatic nerve ligation. Then, ginsenoside Rb1 was intravenously administered continuously by using the continuous injection pump at a dose of 12 µg/day (hereinafter referred to as "S12") or 60 µg/day (hereinafter referred to as "S60") and a rate of 0.25 µl/hour for 4 weeks. Similarly, saline was intravenously administered continuously for 4 weeks as the control group.

### (3) Evaluation of pain

As methods for evaluation of pain, a heat test and a pressure test were used. The following pain evaluations were performed in a quiet room between 9.00 am and 0.00 pm at a room temperature maintained constant.
(a) Heat test
To determine thermal paw withdrawal latency, a heat test was performed by using Plantar Test produced by Ugo Basile (Planter test 7370, Ugo Basile, Italy) according to the method of Hargreaves et al. (Pain, Vol. 32, pp.77-88, 1988). Rats were placed on a plastic table maintained at a temperature of 23 to 24°C and stimulated on the sole by using a thermal stimulator, and withdrawal latency (seconds) was measured. Occurrence of hyperalgesia was determined on the basis of shortening of the latency. The latency was measured 3 times for both the soles always at an interval of 5 minutes, and the average of the results of 6 times in total of measurements was used as a measured value.

(b) Pressure test
To measure the pain threshold for a mechanical stimulus (von Frey withdrawal threshold), the von Frey hair test was performed in the same manner as in the Semmes-Weinstein test (Z. Gesamte Neurol. Psychiat., Vol. 79, pp.324-333, 1922; Perceptual and Motor Skills, Vol. 14, pp.351-354, 1962; The Journal of Hand Surgery, Vol. 3, pp.211-216, 1978), which is clinically performed. A rat was placed in a wire mesh cage having a size of 30 × 35 × 10 cm. Then, the hind limb soles were stimulated 4 times, starting with a thin von Frey filament and gradually changing the filament to thicker ones. When the rat showed withdrawal behavior for all the stimuli of 4 times, the result was determined positive. Five minutes later, the soles were stimulated 4 times in the same manner, starting with a filament thinner by 2 levels than the filament with which the rat showed the positive result for the first time, and stimulation was continued until the rat showed withdrawal behavior for all the stimuli of 4 times. In this procedure, the pain threshold (g) for the pressure stimuli to both the soles was measured twice for each time, and the average of the results obtained in measurements of 4 times in total was used as a measured value. Occurrence of allodynia was determined on the basis of the fact that the rat showed withdrawal behavior with a stimulus weaker than that giving withdrawal behavior before the operation.

### (4) Measurement of α2A-adrenergic receptor mRNA

The spinal cord in the sciatic nerve innervation territory was removed from rats of the sham operation group (n = 7) and rats 3 weeks after the sciatic nerve ligation (each n = 7) which were intravenously administered with saline or 60 µg/day of ginsenoside Rb1 (S60) continuously from 24 hours after the sciatic nerve ligation, and total RNA was extracted by using ISOGEN (Nippon Gene, Tokyo). The extracted total RNA was treated with DNase, and single-stranded cDNA was synthesized by using oligo-dT primers and Moloney murine leukemia virus reverse transcriptase (Life Technologies, Rockville, USA). The sequences of the primers used in this example were as follows.
Rat β-actin:
Sense: 5'-AGAAGAGCTATGAGCTGCCTGACG-3'
Antisense: 5'-TACTTGCGCTCAGGAGGAGCACTG-3'
Rat α2A-adrenergic receptor
Sense: 5'-GCTCGCTGAACCCTGTTATC-3'
Antisense: 5'-TCCCCTCCAAACTGGGTATT-3'

PCR was performed under the following condition using Taq polymerase (Takara, Tokyo). For β-actin, after a reaction at 94°C for 5 minutes, a cycle of heat denaturation at 94°C for 1 minute, annealing at 55°C for 1 minute and 30 seconds and elongation at 72°C for 1 minute and 30 seconds was repeated 25 times. For α2A-adrenergic receptor, after a reaction at 94°C for 2 minutes, a cycle of heat denaturation at 94°C for 1 minute, annealing at 60°C for 1 minute and elongation at 72°C for 1 minute was repeated 30 times. As the internal control, β-actin was used.

### (5) Examination of expression of α2A-adrenergic receptor protein

The spinal cord in the sciatic nerve innervation territory was removed from rats of the sham operation group (n = 3) and rats 3 weeks after the sciatic nerve ligation (each n = 3) which were intravenously administered with saline or 60 µg/day of ginsenoside Rb1 (S60) continuously from 24 hours after the sciatic nerve ligation, added to 10 times in volume of 50 mM phosphate buffer, 100 µM pAPMSF, 0.5% Triton-X and 0.5% SDS, and homogenized. The homogenate was added with a half volume of 6% SDS (+ β-mercaptoethanol), incubated at 99°C for 10 minutes and centrifuged at 15,000 rpm and 4°C for 10 minutes, and the supernatant was used as a sample. Electrophoresis was performed on 10% SDS polyacrylamide, and then the protein was transferred on a polyvinylidine difluoride membrane (Millipore, Bedford, U.S.A.) by using a transblotting apparatus. Western blotting was performed by using anti- α2A-adrenoceptor antibody (ALEXIS BIOCHEMICALS, San Diego, USA) as the primary antibody and anti-rabbit IgG conjugated with alkaline phosphatase (SIGMA Chemicals, St. Louis, U.S.A) as the secondary antibody.
(6) Statistical method
For statistical analysis, one factor ANOVA and post hoc Bonferroni analysis were performed. With P < 0.05, it was determined there was a significant difference.

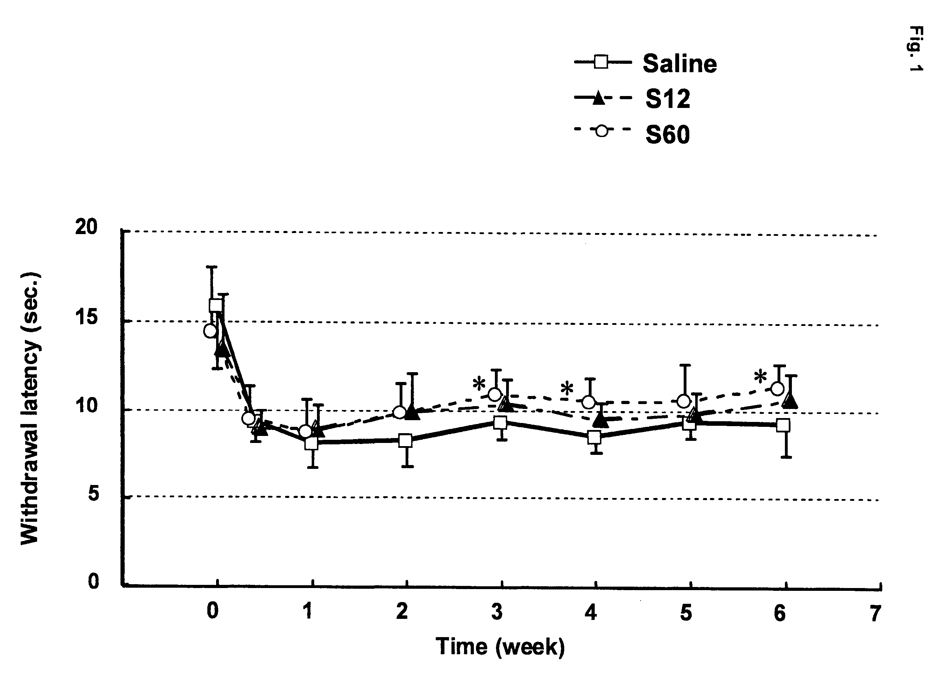

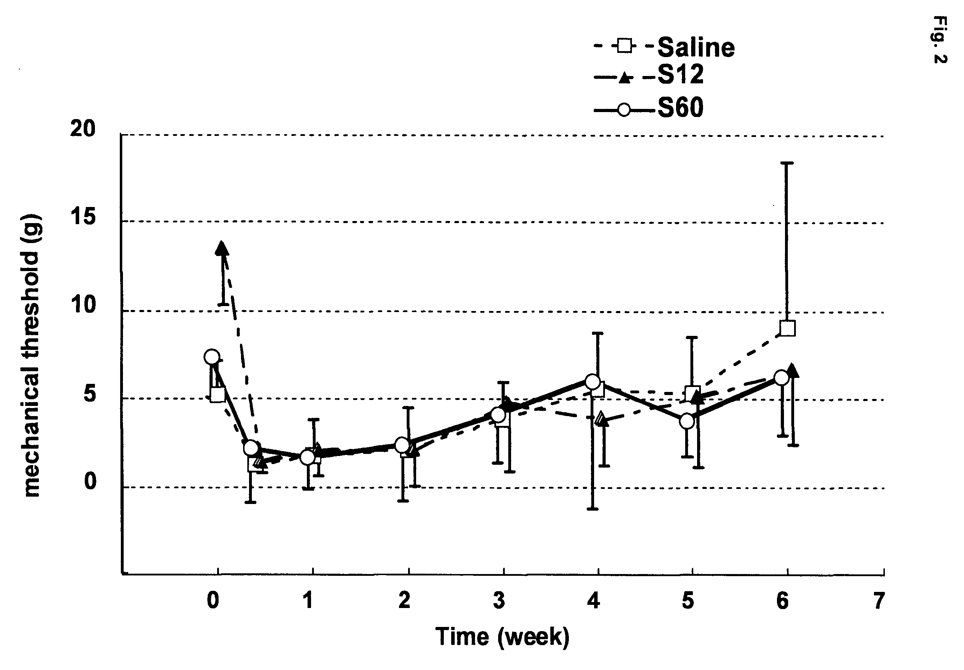

B. Results
The heat test was performed 24 hours after the sciatic nerve ligation, and only rats showing an average latency for both feet of 90% or lower of the same before the operation were used. Animals in a number of 32 in total were arbitrarily divided into 3 groups (10 or 11 animals per group) and used for the experiment. The pressure test and the heat test were performed 3 days and 1, 2, 3, 4, 5 and 6 weeks after the ligation. Although amelioration of allodynia was not observed in comparison of the Rb1-administered group and the control group, improvement of hyperalgesia was observed 3 weeks after the ligation and thereafter. Therefore, when Rb1 was continuously administered for 4 weeks from 24 hours after the ligation, hyperalgesia was significantly relieved 3 weeks after the ligation and thereafter (Figs. 1 and 2).

To elucidate the action mechanism of Rb1, examination was performed by using depletors or blockers against substances reported to be involved in the descending pain signal transmission in the spinal cord, such as serotonin depletors, opioid receptor antagonists, α-adrenergic receptor blockers, and β-adrenergic receptor blockers (Biol. Pharm. Bull., 22-7, pp.691-697, 1999). The heat test was performed 24 hours after the sciatic nerve ligation, and only rats showing an average latency for both feet of 90% or lower of the same before the operation were used. Animals in a number of 48 in total were arbitrarily divided into 8 groups (6 animals per group) to prepare Rb1-administered groups and a control group. A serotonin depletor, opioid receptor antagonist, α-adrenergic receptor blocker and β-adrenergic receptor blocker were each administered 3 weeks after the ligation, the heat test was performed before and after the administration, and the results were compared.

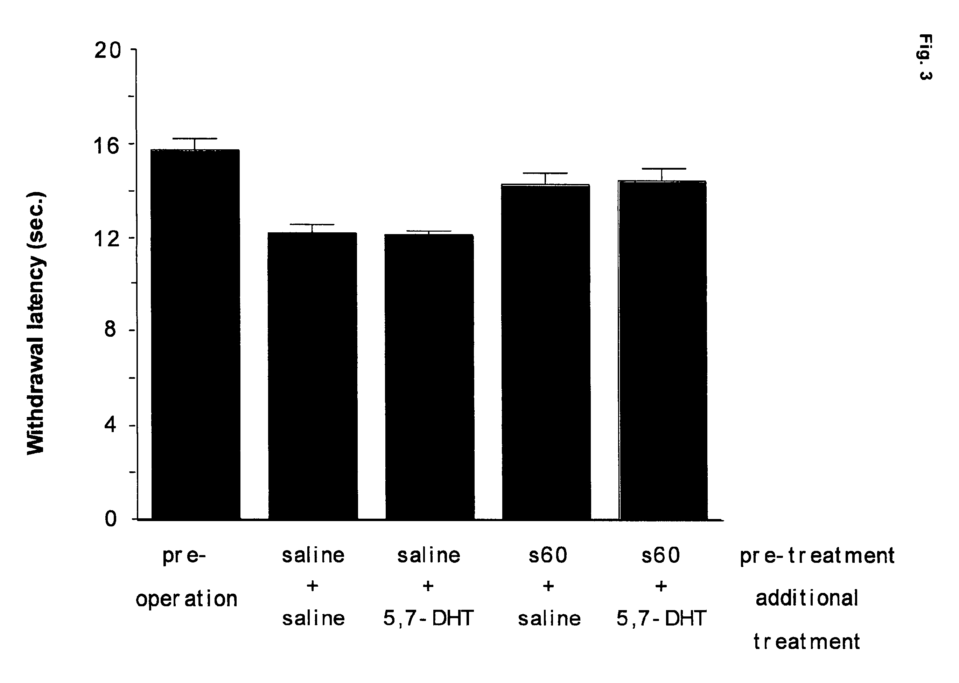

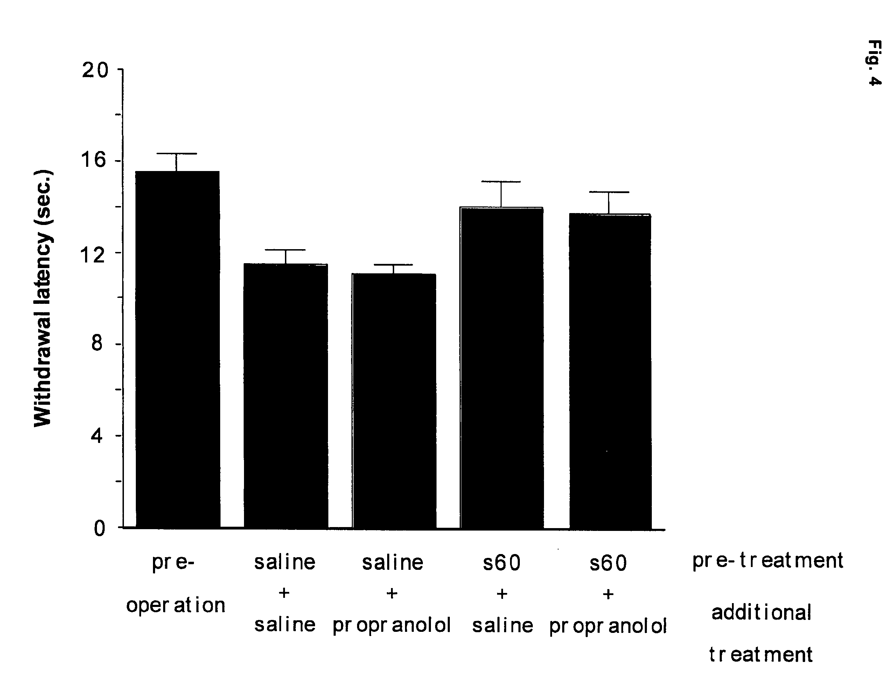

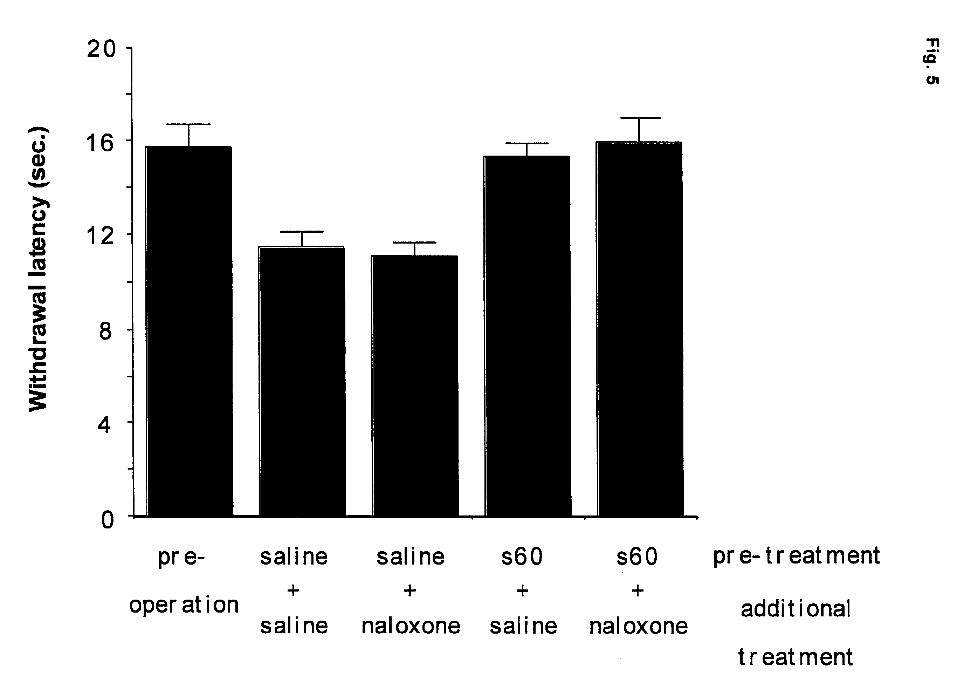

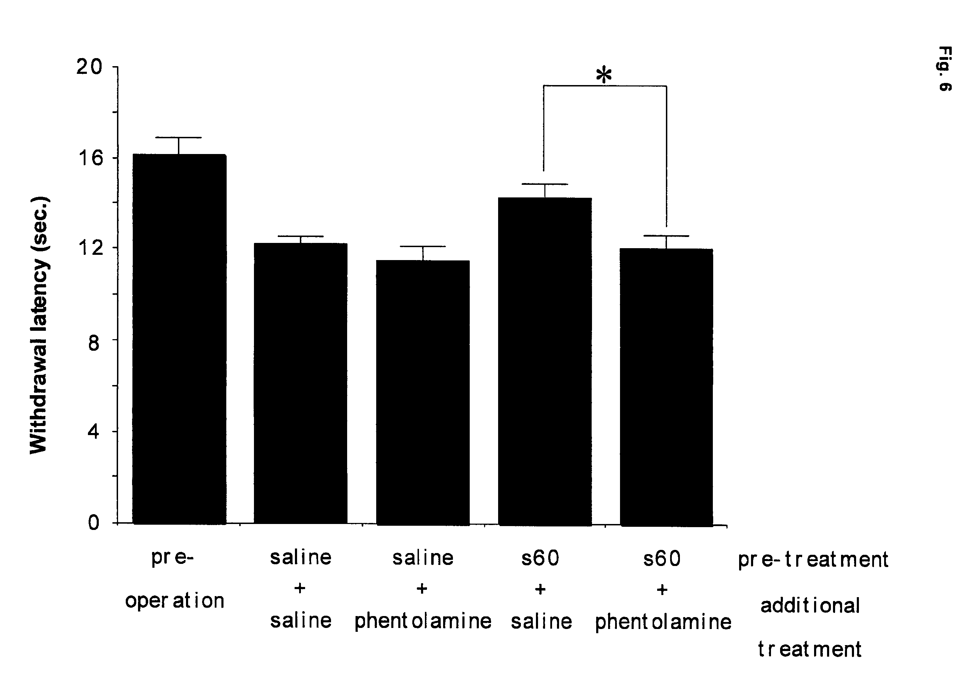

The serotonin depletor, 5,7-dihydroxytryptamine (5,7-DHT, ICN Biomedicals Inc., Ohio, U.S.A), was intradurally administered in an amount of 60 µg 1 week before the measurement, and the heat test was performed 3 weeks after the ligation. Further, the heat test was performed 3 weeks after the ligation, on the following day, 5 mg/kg of the opioid receptor antagonist, naloxone (USP, Rockville, U.S.A), 10 mg/kg of the β-adrenergic receptor blocker, propranolol (SIGMA-ALDRICH Co., St. Louis, U.S.A), and 20 mg/kg of the α-adrenergic receptor blocker, phentolamine (SIGMA-ALDRICH Co., St. Louis, U.S.A), were each subcutaneously injected, and the heat test was performed again one hour later. As a result, no significant difference was observed between the animals administered with 5,7-DHT and not administered with 5,7-DHT in both the Rb1-administered groups and the control group (Fig. 3). Similarly, no significant difference was observed in both the Rb1-administered groups and the control group in the heat tests before and after the administration of propranolol and naloxone (Figs. 4 and 5). However, although no significant difference was observed in the control group in the heat tests before and after the administration of phentolamine, the hyperalgesia suppressing effect of Rb1 was significantly inhibited in the Rb1-administered groups after the administration of phentolamine (Fig. 6). These results suggested that the α-adrenergic receptor was associated with suppression of hyperalgesia by ginsenoside Rb1.

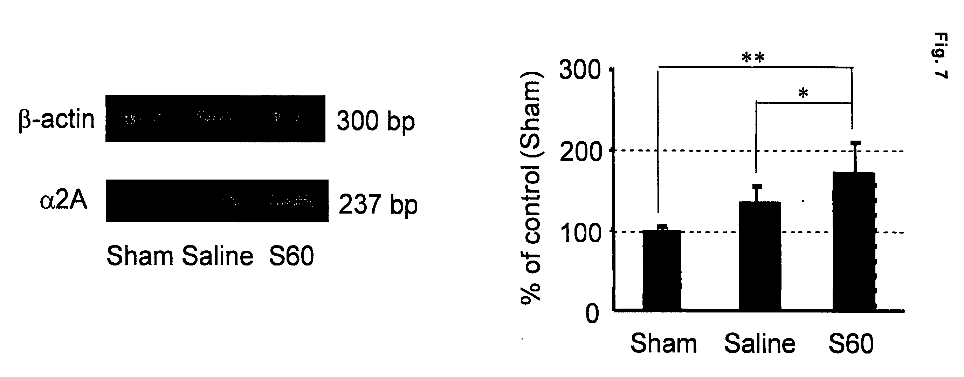

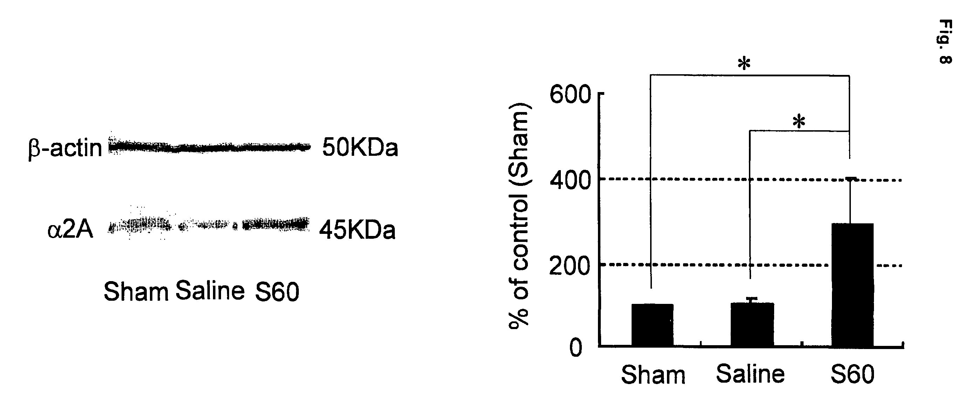

As the association of the α-adrenergic receptor with the hyperalgesia suppressing effect of ginsenoside Rb1 was suggested, changes due to administration of Rb1 in gene expression of the α2A-adrenergic receptor, which is considered to most deeply relate to the analgesic action, in particular, at the spinal cord level among α-adrenergic receptors, was examined by RT-PCR. As a result, the expression level of the α2A-adrenergic receptor mRNA in the spinal cord 3 weeks after the sciatic nerve ligation significantly increased in the Rb1-administered group compared with the control group and the sham operation group (Fig. 7). Similarly, changes in α2A-adrenergic receptor protein expression in the Rb1-administered groups were examined by immunoblot analysis. As a result, the expression level of the α2A-adrenergic receptor protein in the spinal cord 3 weeks after the sciatic nerve ligation significantly increased in the Rb1-administered groups compared with the control group and the sham operation group (Fig. 8).

### Industrial Applicability

The present invention provides a medicament that has superior efficacy for therapeutic and/or prophylactic treatment of pain such as neuropathic pain that occurs after peripheral or central nerve injury.

## Claims

1. A medicament for therapeutic and/or prophylactic treatment of pain, which comprises ginsenoside Rb1 as an active ingredient.

2. The medicament according to claim 1, wherein the pain is chronic pain.

3. The medicament according to claim 1 or 2, wherein the pain is neuropathic pain.

4. The medicament according to claim 3, wherein the neuropathic pain is pain induced by peripheral and/or central nerve injury.

5. A medicament for therapeutic and/or prophylactic treatment of pain, which is an α2A-adrenergic receptor-agonistic medicament comprising ginsenoside Rb1 as an active ingredient.
